# EUROPEAN PATENT APPLICATION

(11) **EP 3 351 093 A1**
(43) Date of publication of application: **25.07.2018**
(21) Application number: 16838142.4
(22) Date of filing: 16.08.2016
(51) Int. Cl.: A01K 45/00

(54) **SYSTEM FOR RECOGNISING THE STAGE OF FERTILE EGGS FROM AN OVOSCOPY STEP AND SORTING SAME, AND METHOD FOR OPERATING THE SUBMODULES OF THE SYSTEM AND MODULES FOR APPLYING SUBSTANCES AND HATCHING**

(30) Priority: 25.08.2015 BR 102015020511
(71) Applicant: CEVA SAÚDE ANIMAL LTDA., 13140-000 Santa Terezinha, Paulínia - SP (BR)
(72) Inventor: Bastos, César da Silva, 13020-060 Campinas - SP (BR)
(74) Representative: Cabinet Becker et Associés
(86) International application number: PCT/BR2016/050197
(87) International publication number: WO 2017/031561

(57) **Abstract**

A system for recognising the stage of fertile eggs from an ovoscopy step and sorting same, and a method for operating the submodules of the system and modules for applying substances and hatching represents an inventive solution in the field of poultry farming, in particular in the sector of poultry breeding, and more specifically in the step of application of vaccines or nutrients or a vaccine-nutrient complex into fertile eggs by intra-ovum injection, and also in the hatching step, on the basis of a module for sorting eggs in the matrix (4), in particular blind matrix (Mz1) of fertile eggs, and of an egg stage sensing module (M41), wherein the data obtained are processed by an egg sorting module (m42), using an application software (d21) which first generates a virtual matrix (Mz2) of the incubation tray, then a matrix (Mz3) that mirrors approved eggs, these data being then processed to feed the substance preparation submodule (m43) and the device set-up module (m44), in order to optimise the operation of the substance application module (m2) and hatching module (m3), respectively, a sorted egg data base submodule (m45) being further defined to feed the module for analysing the quality of the matrices (egg-laying hens) used to develop avian species.

## Description

### TERMINOLOGY

In order to better understand the subject matter disclosed and claimed in the present patent application, the meaning of some terms richly cited in the body of the descriptive report, where:
Egg-candling: is a procedure where it is verified if the eggs were fertilized and if they are not, are removed from the process of creation poultry or similar species.
- Avian species: its concept refers to poultry farming that is the breeding of poultry for food production, especially meat and eggs. Among the species raised on poultry, chicken stands out. On a much smaller scale, birds are also raised such as turkeys, ducks, geese, quails, and ostriches.
- PLC: Programmable Logic Controller is a specialized computer, based on a microprocessor that performs control functions through software developed by the user (each PLC has its own software); and
- HMI: Human Machine Interface.

### APPLICATION FIELD

The present invention patent with title in the epigraph and subject of description and claim in this cartoon, deals with an inventive solution that finds outstanding benefit in the sector of poultry, notably in the sector of poultry reproduction, and more specifically in the stage of application of vaccines and or nutritional or nutritional vaccine complexes infertile eggs, by means of intra-egg ingestion.

More specifically, the invention finds particular utility when applied as an aid to the operation of sub-modules of a module for the application of substances in intra-fertile eggs and hatchery operating module.

### BACKGROUND OF THE INVENTION

In view of the previously defined field of application, the applicant, an expert in the area of breeding and development of embryos for the establishment of poultry species, following detailed studies of the entire classification process, vaccination and/or nutrition and breeding procedures, identified, at the vaccination and/or nutrition stage, a list of emerging needs, of which the egg-laying procedure should be highlighted after delivery of fertile eggs in the intra-egg vaccination/nutrition stage, through a tray in which eggs identified as fertile are deposited, resulting in an egg matrix with different characteristics from a dimensional point of view, of positioning in the accommodation niche in the tray, defective shells, and absence of eggs.

These different characteristics are not perceived by the conventional fertile egg management operating system which guarantees operation of the operational sub-modules of the fertility egg vaccination/nutrition module, namely vaccine/nutrient injection sub-module, disinfection sub-module after vaccination/nutrition, hatcher transfer sub-module.

In tight synthesis, it is possible to state that conventional egg management systems classified as fertile in the candling step are a blind system, characterized by discrete events, that is, with processes in which the variables assume zero values (presence of eggs) or one (absence of egg), for example.

However, an egg management system developed by Pfizer© is anticipated by the state of the art, which is not totally blind, identifying the presence of eggs, but, in turn, it does not classify the egg size or whether there is the presence of broken eggs.

In view of the scenario about the operational logic of a conventional vaccination/nutrition module, the applicant has identified a major need to optimize the operational sub-modules of the vaccination/nutrition module, which, in a more specific way, means of providing an operation where:
a. From the vaccination/nutrition operation perspective: need to optimize the vaccine/nutrient consumption rate carried out by the vaccination/nutrient application sub-module, especially for fertile eggs considered suitable for development until the transfer of fertile eggs, and not only for the identification of the presence or not of eggs in the niches inside the tray derived from the candling module.
b. From the post-vaccination/nutrition disinfection perspective: where it is desired that the sanitizing consumption is optimized in its formulation [concentration], based on the age of the vaccinated fertile eggs population [calculated by the average size of the eggs] with its release of all vaccine/nutrient application devices that effectively had been operational.
c. About the transfer of eggs to hatcher: it is desired the possibility to perform the selective level adjustment during the transfer of the eggs from the incubation tray to the hatchery basket, according to their size, minimizing the occurrence of non-conformities by breaking the already vaccinated and/or nourished fertile egg, maximizing productivity and avoiding the waste of inputs, such as vaccines, nutrients, nutritional vaccine complex and sanitizing product.

### SUMMARY OF THE INVENTION

In accordance with the demand of the invention, the applicant designed a "system for recognizing and classifying the height of fertile eggs derived from the candling stage, applied in operational sub-modules of an intra-egg vaccination/nutrition module" of fertile eggs, provided of novelty associated with inventive activity, since it does not follow in an obvious way other techniques anticipated by the state of the art, conferring advantages from the industrial, commercial and technical points of view.

Furthermore, the "invention" is provided with industrial applicability, being economically feasible and therefore, taking into account the strictness of patentability requirements, notably as a patent of invention, as set forth in the provisions of articles 8 and 13 of Law 9.279.

### BACKGROUND OF THE TECHNIQUE

In order to provide veracity, and to consolidate the explicit context in the topics of the introductory table, an explanation is presented on the state of the art for a vaccination/nutrition module and its main sub-operational modules, where after a critical analysis of these, once evaluated by technicians in the subject, can identify its limiting aspects, thus consolidating the identification of the previously mentioned demand.
a. The architecture of the conventional system: in tight synthesis, selection, vaccination of fertile eggs and dispatch for incubation is performed by the following operational modules:
   a.1 Candling Module: wherein tight understanding the operation of selection of fertile eggs can be made with equipment of a manual or automated nature. For manual equipment, the selection operation is done with the aid of a table that emits light under the eggs and the operator visualizes those that allow greater passage of light classifying them as infertile eggs. In this way, the procedure of removing the infertile eggs from the incubation tray is undertaken;
   a.2 Substance application module: for the purpose of better compression of the present invention, only two sub-modules will be considered to integrate this operation module:
      a.21 Substance injection sub-module: A reservoir for substances (vaccine, nutrient or nutritional vaccine complex) is provided, which in turn, feeds a matrix of injector devices, each provided with punches and needles, an injector device is defined for each egg housing niche defined in the incubation tray. From the operational point of view, once the incubation tray is positioned under the vaccination module, the matrix of injectors is driven, moving down collectively towards the corresponding niches of the incubator tray, where at that time, the computerized system releases a vaccine dose (or nutrients) to the needles; and
      a.22 Injector sanitization sub-module: a reservoir for sanitizing is provided, which in turn feeds the matrix of injector devices used for the injection of substances. From the operational point of view, as the injection of substances is completed, the injector platform retreats or is elevated, distancing the matrix of injectors from the eggs, and then the sanitizing dose for the sterilization of the needles is released; and
   a.3 Fertile egg transfer module: after vaccination of the fertile eggs contained in the incubator tray, they are withdrawn from the incubation basket, and transferred to a hatchery basket by means of a suction cup device arranged in a matrix form, descending simultaneously, and moved by a displacement platform, up to a mean distance, where vacuum is applied to both the suction cups corresponding to the fertile eggs, and to empty housings, where the fertile eggs are "sucked in" by the suction cups, and in vertical movement, are transferred to the hatchery basket, and released from the suction action of the suction cups by accommodating themselves in the niches of that hatchery basket.
b. Identification of problems: for the purpose of better understanding this invention, the critical analysis is limited to the module of application of substances and its injection and sanitization sub-modules, and also to the fertile egg transfer module.

### b1. From the perspective of the substance application module:

b.1 Substance injection sub-module: Vaccine consumption, nutrient or nutritional vaccine complex is performed in a "blind" way, that is, it does not recognize in the matrix of fertile eggs received from the candling module of each hatcher niche, the status of each fertile egg thus leading to selection of all niches for the immunization/nutrition of the embryo, converging on unnecessary input consumption (vaccine and or nutrient) since its matrix of injectors is totally activated;
   Searches in patent database disclose US Pat. No. 7,430,987 B2 entitled "AUTOMATIC EGG VACCINATOR", which comprises an automated system controlled by a PLC (programmable logic controller), using injectors provided with electronic contact sensor for signalizing abrupt empty egg spaces, and/or the injection of vaccine avoiding their waste.
   Although the automatic vaccination module of the US patent document No. 7,430,987 B2 discloses mechanisms that truncate the ejection of substances (vaccines, nutrients or nutritional vaccine complex), however, even in empty niches, the set of punchers and needle are exposed out of the body of the injection device, creating a contamination risk condition of the same.
   Another aspect to be considered is that this same prior art document is limited to detect only presence or non-presence of egg, that is, its operational logic is framed in discrete events (SEDs), that is, with processes in which variables assume values 0 (egg present) or 1 (egg not present).
   This aspect is too restrictive, considering that within the universe of an incubator tray, which forms a matrix of fertile eggs, this selective vaccination system does not identify the condition in which the eggs in the niches show themselves, for example, egg size, if the egg is lying down or even broken, thus compromising the desired customization of the substance injection operation (vaccines, nutrients or nutritional vaccine complex).
b2. Injector sanitization sub-module: are aware that the level of contamination is directly proportional to the egg size delivered by the bird, and in turn, the size of the egg is directly related to the age of the bird, which, in an allusive form, means that while larger eggs are derived from matrices of older birds, smaller eggs are derived from younger bird matrices.

Given this condition, a careful observation of the conventional injector sanitization sub-module procedure reveals that, in the prior art, the egg size/sanitizing concentration ratio was not considered.

This time, the injectors sanitization sub-module also operates in a "blind" way because it does not recognize in the matrix of vaccine/nutrient injector devices, those in which the vaccination/nutrition operation was effectively performed, releasing the sanitizer to all the vaccination/nutrition devices.

In turn, because it is a blind operation, it also repeats its operational logic being framed in discrete events (SEDs), that is, with processes in which variables assume 0 values (sanitize) or 1 (not sanitize).

From the point of view of the mandatory sanitization of the punch and needle components, the calculation of the sanitizer concentration is carried out empirically, through the average batch of eggs is framed within a concentration range, where that batches can be classified as batch of large eggs, batch of medium eggs and batch of small eggs, and the measured sanitizing concentration is applied to all injecting devices. However, this concentration can vary according to the classification of the egg lot as P, M or G).

### b3. From the perspective of the transfer of eggs to the hatchery basket:

Due to the difference in egg height, which may be basically large, medium and small, in this case, because the displacement of the set of suction cups is defined by a mean measure, there are recurrent problems such as:
- In the case of batch of large eggs, the suction cup moves forward on the egg upper surface while the suction cup platform with the "sucked" eggs moves forward for dumping them in the hatchery basket, generating an excess of compression, which can lead to egg-shell rupture, and consequently, rendering unusable an already vaccinated or nourished embryo, causing productivity losses and input costs (vaccine, nutrient, nutritional vaccine complex, and sanitizing solution), thus increasing the cost of the operation;
- In the case of small eggs, the suction cup also moves forward on the upper egg surface when the suction cup platform with the "sucked" eggs moves forward to dump them in the hatchery basket; however, the egg remains far from the niche bottom, and when becomes free from the suctioning action of the suction cup, it can lead to the rupture of the eggshell by free fall, and, consequently rendering unusable an embryo already vaccinated or nourished, causing both productivity and input losses (vaccine, nutrient, nutritional vaccine complex, and sanitizing solution), thus increasing the operational costs.

In an allusive form, the reason for the reported problems lies in the lack of a selective control that considers the actual average egg size to transfer to the hatcher niches, where the adjustment is collective according to the batch classification as batch of large eggs, batch of medium eggs or batch of small eggs, making the fertile and/or nourished fertile egg transfer module to work with a standard Cartesian Z-axis displacement, which is not suitable for transporting fragile eggs whose integrity is broken by pressure or impact.

### OBJECT OF THE INVENTION

a. Objective: it is an object of the present invention to eliminate all of the limitations disclosed for the studied operating modules where:
   a1. From the perspective of the substance application module:
      a.11 Substances injection sub-module: starts receiving distinctive information about the classification of the fertile eggs present in the egg matrix from candling, wherein for making this condition feasible, the invention has a distinctive character because it provides the identification of the classification of each egg in each niche of the egg matrix, from of the height recognition of these eggs, thus generating an egg matrix with the desired "virtual view" in which each egg niche is identified as being "large egg", "medium egg" and "small egg" allowing mixing with dosage of appropriate sanitizing concentration for each of these egg size classes, minimizing any possibility of contamination of the next batches of eggs that will receive injections of substances (vaccines, nutrients or nutritional vaccine complex).
      a.12 Injector sanitization sub-module: starts receiving distinctive information about the classification of the fertile eggs present in the egg matrix from candling, and in order to make this condition feasible, the invention has a distinctive character because it provides the identification of classification of each egg in each niche of the egg matrix, from the recognition of the height of these eggs, thus generating an egg matrix with the desired "virtual view" of the substance injection sub-module, and making possible to obtain the reliable value of the variable "average egg size" present in the incubation tray.
   a2. From the perspective of the level of transfer of eggs to the hatcher: the sub-module of transference for hatcher begins to receive information from the fertile egg classification management system regarding its height, and automatically begins to recognize the height of each egg in each niche of the matrix of fertile eggs by the size of the egg, Large (L), Medium (M) or small (S), thus generating an egg matrix with the desired "virtual vision" and making possible to obtain the reliable value of the variable "average egg size" present in the incubation tray.

### DESCRIPTION OF THE FIGURES

To complement the present description in order to obtain a better understanding of the features of the present invention, and in accordance with a preferred practical embodiment thereof, the attached description is accompanied by a set of drawings, wherein:
Fig. 1 is an illustrative block diagram representation of the macro-architecture of a conventional fertility egg selection, vaccination, sanitization, and transfer system, showing operational modules and sub-modules;
Fig. 2 is an illustrative block diagram representation of the macro-architecture of a conventional system of selection, vaccination, sanitization, and transfer of fertile eggs, evidencing the application of the unprecedented recognition system and height classification of fertile eggs from the candling stage;
Fig. 3a is a detailed representation of the unprecedented height recognition and classification system of fertile eggs from the candling stage, with its operational sub-modules;
Fig. 3b is a detailed representation of the unprecedented height recognition and classification system of fertile eggs from the candling stage, with its operational sub-modules, showing the devices embedded in each one, as well as illustrating the technical effect obtained in the sub-modules of substance application and post-vaccinated/nourished egg transfer modules;
Fig. 4 is an illustrative representation of the elevation of avian species selection, vaccination, and breeding equipment, evidencing the installation of the egg height capture module of the height recognition and classification system of fertile eggs;
Fig. 5 is an illustrative representation in front view of the selection- vaccination and breeding equipment of avian species, showing the installation of the sub-module for catching the height of eggs and the fertile egg classifier sub-module of the recognition system and classification of height of fertile eggs;
Fig. 6 is an illustrative representation in elevation view of an example of a disposition of the fertile egg blind matrix from the candling module, showing the recognition only of the presence of eggs or no eggs in the niches of the incubation tray;
Fig. 7 is an illustrative representation (example) in elevation view of disposition of the virtual egg classification matrix for each niche of the incubation tray;
Fig. 8 is an illustrative elevation view of a layout example of a approved egg mirror matrix that the hatcher module begins to "verify";
Fig. 9a is an illustrative depiction of a hatchery basket of the fertile egg transfer module, in a condition where it is receiving eggs, and evidencing the identified problem of no egg transfer adjustment according to its size, and
Fig. 9b is an illustrative depiction of a hatchery basket from the improved fertile egg transfer module, in a condition where it is receiving eggs evidencing the existence of egg transfer adjustment according to their size.

### DETAILED DESCRIPTION

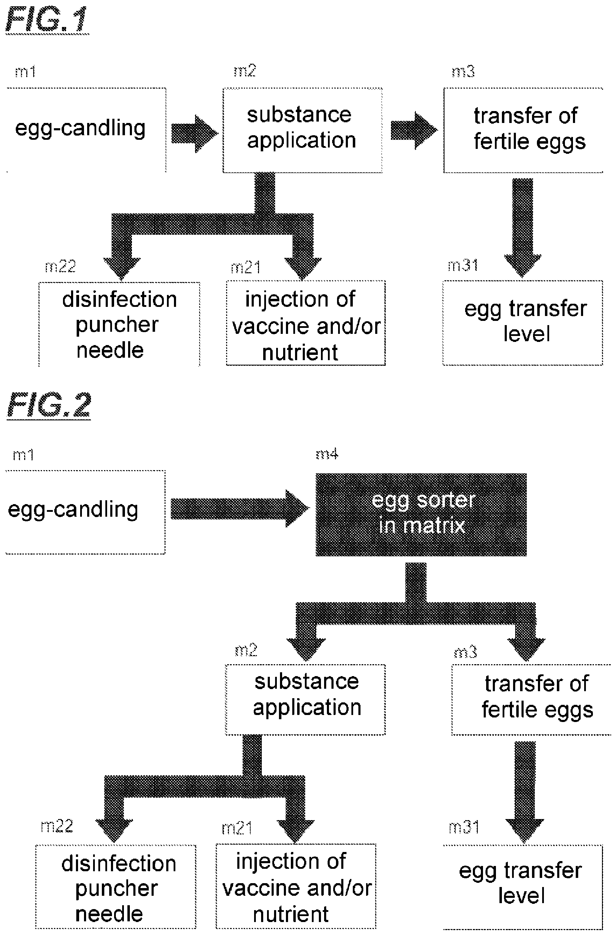

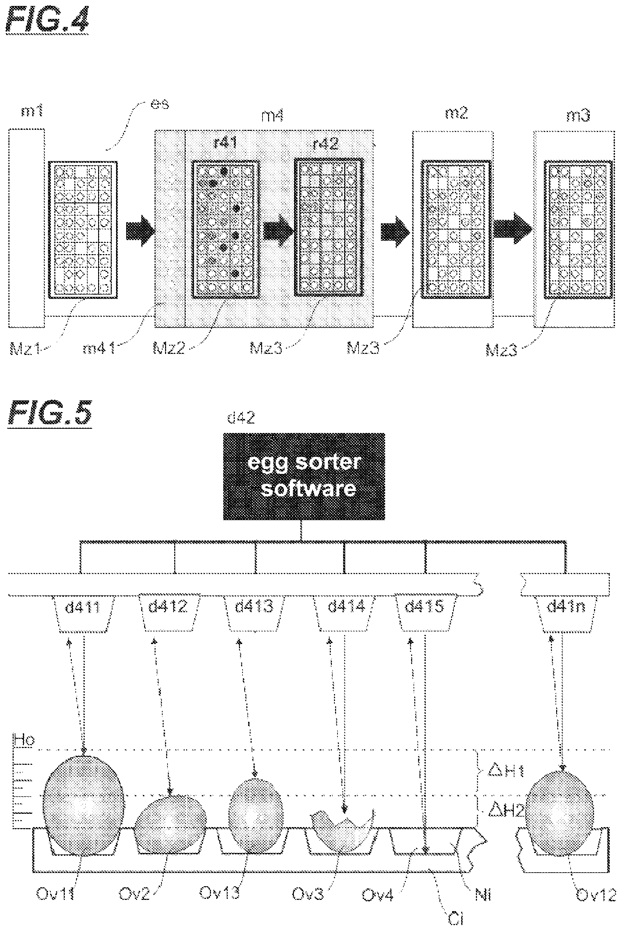

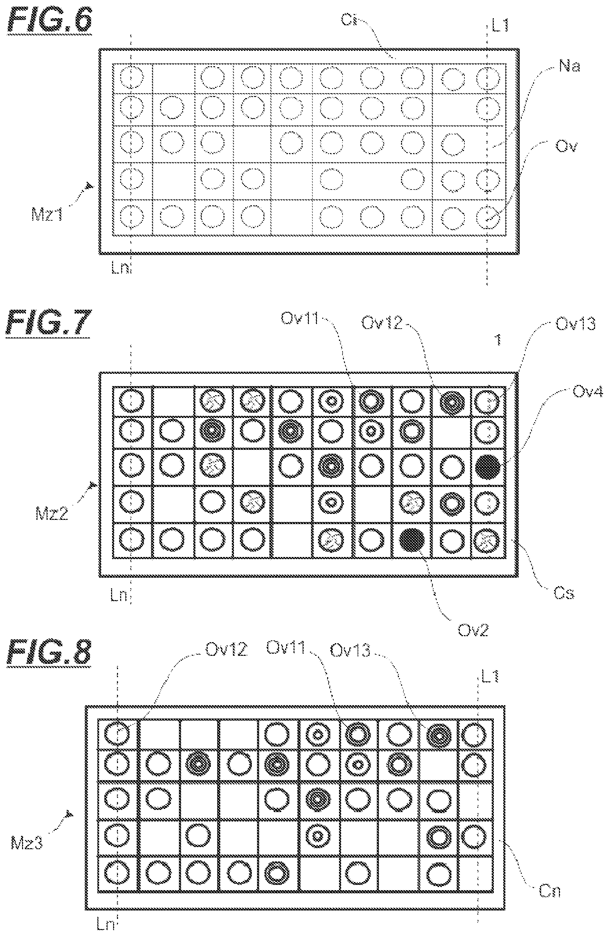

The following detailed description should be read and interpreted with reference to the presented drawings, representing the state of the art for selection, application of conventional substances and hatcher, as well as the architecture and operational logic boarded by the system of recognition and inventive classification of height of fertile eggs from the candling stage, and hence is not intended to limit the scope of the invention, this is limited only to the explicit in the context of the claim.
a. State of the art: as shown in Fig. 1 the macro-architecture of a system for selection, application of substances and hatcher is formed by the following modules:
- Candling module (m1), for the purpose of the present invention, only the incubation tray (Ci) defined in this carton as a fertile egg blind matrix (Mz1) will be mentioned, where the conventional system of selective application of substances from intra fertile eggs, by which only the condition of present egg (Ov) and not egg (Na) is recognized, as evidenced in Figs. 4 and 6, the basis of understanding being the technical effect obtained with the implantation of the recognition and inventive classification system of the height of fertile eggs;
- Substance application module (m2): for the purpose of the present invention among its operational sub-modules, only those benefiting from the inventive fertile egg height recognition and classification system, namely:
   - vaccine/nutrient injection sub-module (m21), where vaccine and/or nutrient is delivered by injector devices provided with needles;
   - puncher/needle disinfection sub-module (m22), where sanitizing is applied to the needles from each injector device.

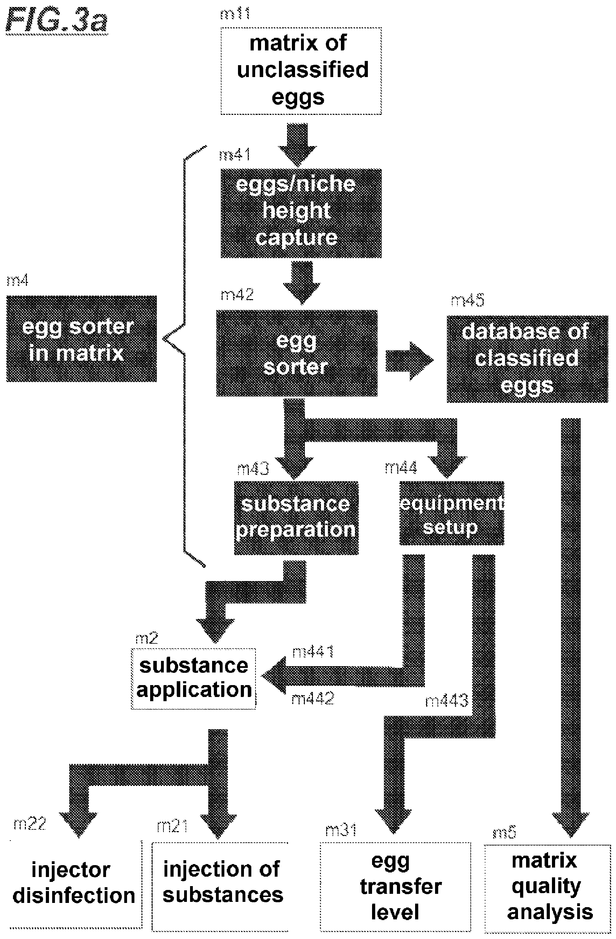

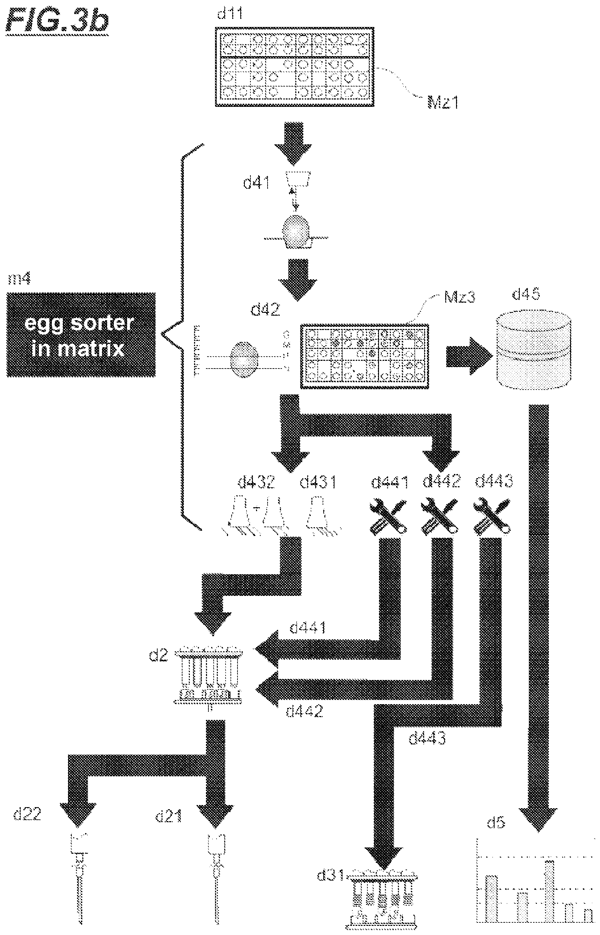

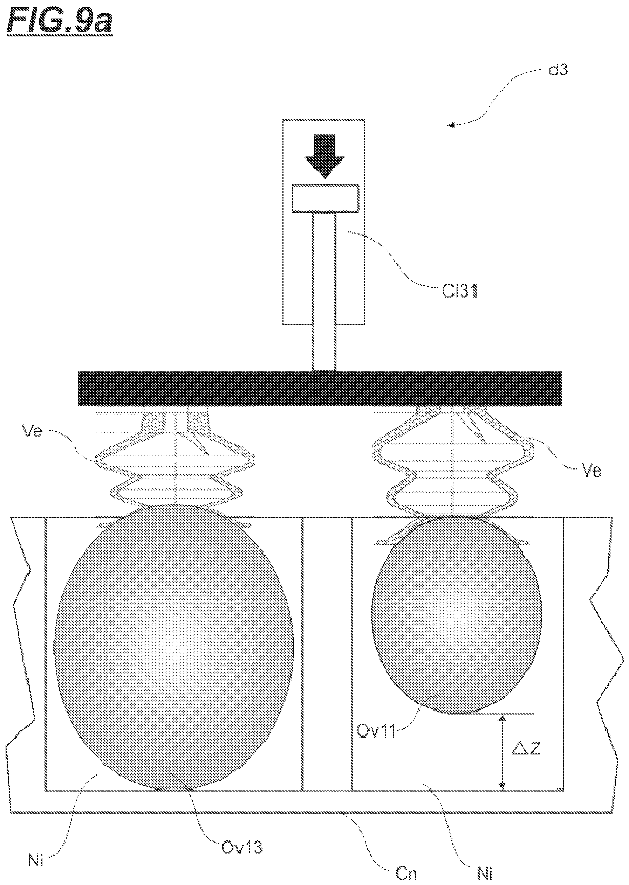

Egg transfer module (m3) for the purpose of the present invention among its operational sub-modules, only the one that benefits from the inventive fertile egg height recognition and classification system was elected, namely the transfer level sub-module (m31);
The conventional transfer level sub-module (m31) is properly described through Fig. 9a, as it can be seen, this is formed by a single displacement cylinder (Ci31), which promotes the simultaneous and standard displacement of the suction cups matrix (Ve), which in turn come into contact and suck the upper end of the vaccinated/nourished eggs, being as described in the topic of fundamentals of the technique, being evident there is a height difference (ΔZ) at the bottom of a large egg (Ov11) relative to the bottom of a small egg (Ov13), which can cause problems of cracking the shells of large (Ov11) and small (Ov13) eggs, due too much compression of the suction cup (Ve) or free fall of the loose egg, respectively.
b. From the inventive technique point of view: as shown in Fig. 2, is introduced the unprecedented egg sorter module in the matrix (4), evidencing its connection and interference in the operating system of the substance application module (m2), and also in the hatching module (m3);
Further details of the egg sorter module in the matrix (4) are revealed by the block diagram of Figs. 3a and 3b, which is defined by the following operating sub-modules:
b.1 sub-module for capturing height of the eggs/niche (m41), has the function of promoting an individualized capture of the height (Ho) of each egg (Ov) deposited in each niche (Ni) of the fertile egg blind matrix (Mz1) of the incubation tray (Ci), which is conveyed by means of a mat (Es) towards the substance application module (m2), wherein the height reading device (d41), formed by a plurality of distance sensors (d411), (d412) (d413), ... (d41n), see Figs. 4 and 5 with any known embedded technology, for example, an analog sensor, see Fig. 5, promotes scanning readings of the condition of each niche (Ni) in the plurality of lines (L1), (L2), (L3), ... (Ln) of the fertile egg blind matrix (Mz1), as shown in Fig. 5;
b.2 Egg sorter sub-module (m42): where for each niche (Ni) reading, of the plurality of lines (L1), (L2), (L3), ... (Ln) of the fertile egg blind matrix (Mz1) an application software (d42) performs the first egg-sorting routine (r41) by the obtained height (Ho), as shown in Figs. 4 and 5, wherein a preferred embodiment can be represented by Table 1.

**Table 1 Egg-sorting after data capture (Ho)**

| **Symbol** | **Reference** | **Egg classification (Ov)** |
|---|---|---|
| 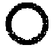 | Ov11 | Large egg type, indicates that it is an old age avian species |
| 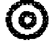 | Ov12 | Medium egg type, indicates that it is a middle age avian species |
| 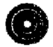 | Ov13 | Small egg type, indicates that it is an avian species of young age |
| 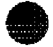 | Ov2 | Laying down egg, out of position in niche |
| 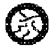 | Ov3 | Broken egg (broken, cracked, ...) |
| | Ov4 | Absence of egg in niche (no egg) |

At the end of the execution of the first routine (r41), the application software (d42) has defined the virtual matrix of the incubation tray (Mz2), as evidenced by Fig. 7.
Once eggs have been classified according to Table 1, the same application software (d42) executes the second classification routine (r42) on which eggs to consider to be treated in the substance application module (m2) and hatching module (m3), where it still has the height as reference, such sieving is defined by the classification of eggs as large egg (Ov11), medium egg (Ov12), small egg (Ov13), laying down egg (Ov2), broken egg (Ov3) and absence of egg (Ov4), respectively, in two lanes of execution, as evidenced in Fig. 5, namely:
- the application execution range of substances and transfer of eggs (ΔH1); wherein in this range of height, only the eggs suitable for the sequence of the breeding cycle are framed, regardless which eggs are classified as a large egg (Ov11), medium egg (Ov12) and small egg (Ov13).
- a application non-execution range of substances and transfer of eggs (ΔH2), as evidenced in Fig. 5.

At the end of the execution of the second routine (r42), the application software (d42) of the egg sorter sub-module (m42) has defined the approved egg mirror matrix (Mz3) as shown in Fig. 8, where the only considered niches (Ni) are those classified in the application execution range of substances and transfer of eggs (ΔH1); furthermore, the sizes of the eggs in each considered niche (Ni) are defined.
Finally, the application software (d42) recognizes the height of each large egg (Ov11), medium egg (Ov12) and small egg (Ov13) in each niche (Ni) of the approved egg mirror matrix (Mz3), obtaining the average size/batch [Lot] for each loading of that matrix, which can be treated as a batch of large eggs [Lot11], batch of medium eggs [Lot12] and batch of small eggs [Lot13].
b.3 Substance preparation sub-module (m43): taking as reference the approved egg mirror matrix (Mz3), the following calculations are performed:
b.31 Calculation of the amount of vaccine/nutrient (m431): in which the same application software (d42) with data on the average size/batch [Lot] in the approved egg mirror matrix (Mz3) prepares the customized amount of vaccine/nutrient (d431) for the egg roll in the cited matrix which will be vaccinated/nourished;
b.32 Calculation of the amount and concentration of the sanitizing solution mixture (m432): namely, a solution based on sodium hypochlorite, where the same application software (d42) with data on the average size/batch [Lot] in the approved egg mirror matrix (Mz3) calculates the customized amount of the chlorine-based solution (d432) for the egg roll in the said matrix, and also with customized concentration that will be used in post-vaccination disinfection, for use of the substance application module (m2), specifically for the injector disinfection sub-module (m22), i.e. for the needle component of the injector devices (d21); see Figs. 3a and 3b;

Whereas the concentration value of the solution of sodium hypochlorite lies in the range of 3,500-8,000 ppm, the concentration is higher as the average height of the eggs increases.
b.4 Equipment setup sub-module (m44): based on the approved egg mirror matrix (Mz3), the following equipment is set-up:
b.4.1 Set-up of the substance application sub-module (m441): wherein the same application software (d42) having as its reference the approved egg mirror matrix (Mz3), activates in the substance application module (m2) only the needles of the injector devices (d21) corresponding to the active coordinates of the matrix (Mz3), and then release the customized amount of vaccine/nutrient (d431) to the substance application module (m2), specifically to the substance injection sub-module (m21), i.e., for the needle component of the injector devices (d21); see Figs. 3a and 3b;
b.4.2 Setup of the sanitizer application sub-module (m442): where the same application software (d42) having as reference the approved egg mirror matrix (Mz3), activates the delivery of the customized amount of the chlorine-based solution (d432) to the substance application module (m2), specifically to the substance injection sub-module (m21), and more specifically to the needles of all the injector devices (d21), see Figs. 3a and 3b;
b.4.3 Setup of the egg transfer module (m443): in which the same application software (d42) having as reference the average size/batch [Lot], which can be classified as large batch, medium batch and small batch, establishes a displacement value (ΔZ11), (ΔZ12) and (ΔZ13) respectively, this value is then sent as signal to activate the egg transfer level sub-module (m31), causing the set of suction devices (d31) to be moved through the action of the sensor [d31], according to the calculated average size/batch [Lot] of eggs, as shown in Figs. 3a and 3b.

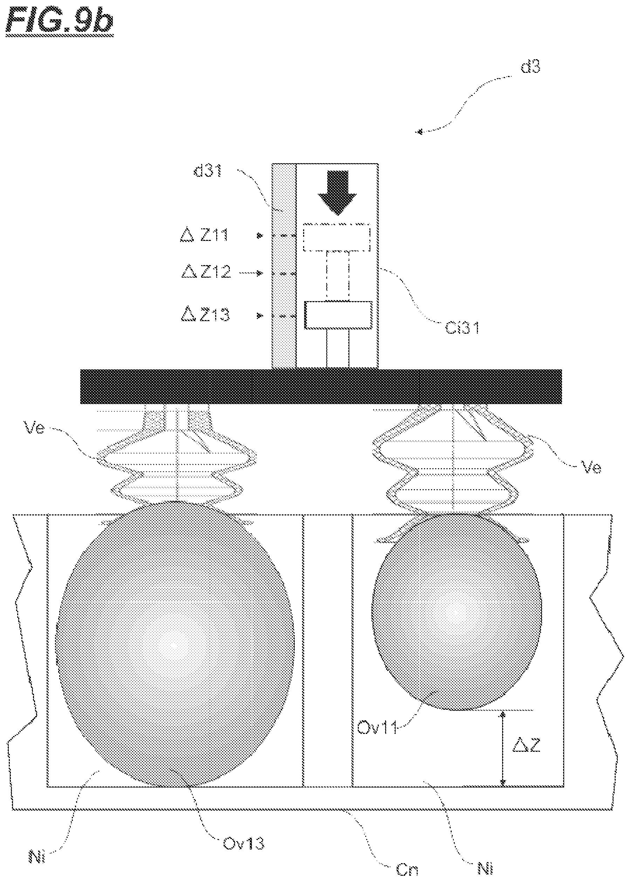

b.5 Database sub-module (m45): as shown in Figs. 3a and 3b, wherein the application software (d42) sends data from each virtual matrix of the incubation tray (Mz2), see Fig. 7, to a database sub-module (m45), which stores them in hardware (d45), where such data on egg classification, according to table 1, is sent to the matrix quality analysis module (m5), in this case, the matrix is understood to be the poultry species that has been treated and developed in the poultry breeding cycle, and this information is processed in order to obtain a quality report from each egg supplier.
c. Improvement in egg transfer module (m3): specifically, its transfer level sub-module (m31) was improved as seen in Fig. 9b, wherein as can be observed, is composed by a dedicated displacement cylinder (Ci31) having a motion sensor (d31) associated with it, which in turn receives the batch size information of the [Lot] eggs through the application software (d42), as large egg batch (Lot11), medium egg batch (Lot12) and small egg batch (Lot13), adjusting for the respective displacement (ΔZ11), (ΔZ12) and (ΔZ13) of the dedicated displacement cylinder (Ci31), which minimizes the height difference (ΔZ) of the eggs relative to the bottom of each niche (Ni) of the hatchery basket (Cn).

The choice of the preferred embodiment of the subject invention in this carton, described in this detailed section, is only provided as an example. Changes, modifications, and variations can be made to any other embodiments of the egg recognition and classification system by their height relative to the bottom of niches of a blind matrix of fertile eggs transported in an incubator tray from the candling module, in which such changes may be designed by those skilled in the art, notably experts in the field of reproduction of poultry species, without however departing from the objective disclosed in the present patent application, which is exclusively defined by the appended claims.

It is seen from what has been described and illustrated, that THE SYSTEM FOR RECOGNIZING AND CLASSIFYING THE HEIGHT OF FERTILE EGGS FROM THE "CANDLING" STAGE claimed herein conforms to the norms governing the invention patent in the light of the Industrial Property Law, and as a consequence deserves the respective privilege.

## Claims

1. System for recognizing and classifying fertile egg height from the candling stage, wherein a macro architecture of a selection system, application of substances and hatcher are formed by a candling module (m1) providing a fertile egg blind matrix (Mz1) that is perceived by a substance application module (m2), which in turn, has a vaccine/nutrient injection sub-module (m21) and a puncher/needle disinfection sub-module (m22), an egg transfer module (m3) showing a transfer level sub-module (m31), formed by a plurality of suction cups (Ve) corresponding to a niche matrix (Ni) of the incubator tray (Ci) and a hatchery basket (Cn), wherein immediately after the candling module, an egg sorter module was introduced in the matrix (4) **characterized by** being composed of a sub-module for capturing height of the eggs/niche (m41) in the form of a height reading device (d41), formed by a plurality of distance sensors (d411), (d412), (d413), ... (d41n), acting on each niche (Ni) of the plurality of lines (L1), (L2), (L3) ... (Ln) of the fertile egg blind matrix (Mz1); an egg sorter sub-module (m42) in the form of an application software (d42) which feeds a substance preparation sub-module (m43) which has as reference the approved egg mirror matrix (Mz3) performs calculation of the amount of vaccine/nutrient (m431) and calculation of the amount and concentration of the sanitizing solution mixture (m432); an equipment setup sub-module (m44) also being fed by the egg sorter sub-module (m42) having as reference the approved egg mirror matrix (Mz3) and performs the setup of the substance application sub-module (m441), the setup of the sanitizer application sub-module (m442) and the setup of the hatching module (m443); a database sub-module (m45) is also provided in the form of a database (d45) also fed by the egg sorter sub-module (m42), sending data to a matrix quality analysis module (m5), wherein the egg transport module (m3) has its transfer level sub-module (m31) composed of a dedicated displacement cylinder (Ci31), to which is linked a motion sensor (d31).

2. System for recognizing and classifying fertile egg height from the candling stage according to claim 1, wherein the sub-module for capturing height of the eggs/niche (m41) of the height reading device (d41), formed by a plurality of distance sensors (d411), (d412), (d413), ... (d41n), is **characterized in that** it is a distance sensor of the analog type.

3. System for recognizing and classifying fertile egg height from the candling stage and operational procedure of its sub-modules according to claim 1, wherein the egg sorter sub-module (m42) in the form of an application software (d42) has its operational process **characterized in that** it executes a first egg classification routine (r41) by the obtained height (Ho), by defining a virtual matrix of the incubation tray (Mz2), and a second classification routine (r42) on which eggs to consider, by defining an approved egg mirror matrix (Mz3).

4. System for recognizing and classifying fertile egg height from the candling stage and operational procedure of its sub-modules according to claim 3, wherein the first egg-sorting routine (r41) for the obtained height (Ho) has the virtual matrix of the incubation tray (Mz2) is **characterized by** containing classification of the niches (Ni) as large egg (Ov11), medium egg (Ov12), small egg (Ov13), laying down egg (Ov2), broken egg (Ov3) and absence of egg (Ov4).

5. System for recognizing and classifying fertile egg height from the candling stage and operational procedure of its sub-modules according to claim 3, wherein the niches (Ni) as large egg (Ov11), medium egg (Ov12), small egg (Ov13), laying down egg (Ov2), broken egg (Ov3) and absence of egg (Ov4) are classified by a second routine (r42) in which eggs are **characterized by** defining an application execution range of substances and transfer of eggs (ΔH1), wherein the framed niches (Ni) are classified as large egg (Ov11), medium egg (Ov12) and small egg (Ov13), and an application non-execution range of substances and transfer of eggs (ΔH2), wherein the framed niches (Ni) are classified as laying down egg (Ov2), broken egg (Ov3) and absence of egg (Ov4).

6. System for recognizing and classifying fertile egg height from the candling stage and operational procedure of its sub-modules according to claims 3 and 5, wherein the approved egg mirror matrix (Mz3) is **characterized by** considering only the niches (Ni) classified as large egg (Ov11), medium egg (Ov12) and small egg (Ov13).

7. System for recognizing and classifying fertile egg height from the candling stage and operational procedure of its sub-modules according to claim 6, wherein for the height values such as large egg (Ov11), medium egg (Ov12) and small egg (Ov13), the application software (md12) calculates the average size/batch [Lot] for each load of that matrix, which can be treated as batch of large eggs [Lot11], batch of medium eggs [Lot12] and batch of small eggs [Lot13].

8. System for recognizing and classifying fertile egg height from the candling stage and operational procedure of its sub-modules according to claim 1, wherein in the substance preparation sub-module (m43), having as reference the approved egg mirror matrix (Mz3), the calculation of the amount of vaccine and/or nutrient (m431) is **characterized in that** the application software (d42) with data on the average size/batch [Lot] in the approved egg mirror matrix (Mz3) prepares the customized amount of vaccine/nutrient (d431) for the egg roll considered in the approved egg mirror matrix (Mz3) which is to be used by the substance injection sub-module (m21) of the substance application module (m2), i.e. to the needle component of the injector devices (d21).

9. System for recognizing and classifying fertile egg height from the candling stage and operational procedure of its sub-modules according to claim 1, wherein in the substance preparation sub-module (m43), having as reference the approved egg mirror matrix (Mz3), the calculation of the amount and concentration of the sanitizing solution mixture (m432) which is specifically a solution based on sodium hypochlorite, is **characterized by** the application software (d42) with data on the average size/batch [Lot] in the approved egg mirror matrix (Mz3) with this value to calculate the customized amount of the chlorine-based solution (d432) and also with customized concentration that will be used by the injector disinfection sub-module (m22) of the substance application module (m2), i.e., for the needle component of the injector devices (d21).

10. System for recognizing and classifying fertile egg height from the candling stage and operational procedure of its sub-modules according to claim 8, wherein the concentration value of the solution based on sodium hypochlorite is **characterized in that** it is situated in the range of 3,500 to 8,000 ppm, the concentration being higher as the average height of the eggs increases.

11. System for recognizing and classifying fertile egg height from the candling stage and operational procedure of its sub-modules according to claim 1, wherein for the equipment setup sub-module (m44) having as reference the approved egg mirror matrix (Mz3), the setup of the substance application sub-module (m441) has an operating procedure **characterized in that** the application software (d42) having as reference the approved egg mirror matrix (Mz3), activates in the substance application module (m2) only the needles of the injector devices (d21) corresponding to the active coordinates of the matrix (Mz3), and then release the customized amount of vaccine/nutrient (d431) to the substance application module (m2), specifically to the substance injection sub-module (m21), in their injector devices (d21).

12. System for recognizing and classifying fertile egg height from the candling stage and operational procedure of its sub-modules according to claim 1, wherein for the equipment setup sub-module (m44) having as reference the approved egg mirror matrix (Mz3), setup of the sanitizer application sub-module (m442) is **characterized in that** the application software (d42) having as reference the approved egg mirror matrix (Mz3) activates the delivery of the customized amount of the chlorine-based solution (d432) to the substance application module (m2), specifically to the substance injection sub-module (m21), and more specifically to the needles of all the injector devices (d21).

13. System for recognizing and classifying fertile egg height from the candling stage and operational procedure of its sub-modules according to claim 1, wherein for the equipment setup sub-module (m44) having as reference the approved egg mirror matrix (Mz3) the setup of the egg transfer module (m443) is **characterized in that** the application software (d42) having as reference the average size/batch [Lot], which can be classified as large batch, medium batch and small batch, establishes a displacement value (ΔZ11), (ΔZ12) and (ΔZ13) respectively, this value being sent as signal to activate the egg transfer level sub-module (m31), causing the set of suction devices (d31) to be triggered, according to the calculated egg batch average size [Lot].

14. System for recognizing and classifying fertile egg height from the candling stage and operational procedure of its sub-modules and modules for application of substances and hatcher according to claim 1, wherein an improved transfer level sub-module (m31) is formed by an operational procedure **characterized in that** the application software (d42) having as reference the average size/batch [Lot], sends this information to the motion sensor (d31) by taking the displacement roller (Ci31) to an offset (ΔZ11), (ΔZ12) and (ΔZ13) for average size/batch [Lot] corresponding to large egg (Ov11), medium egg (Ov12) and small egg (Ov13) eliminating the height difference (ΔZ) of the eggs relative to the bottom of each niche (Ni) of the hatchery basket (Cn).

15. System for recognizing and classifying fertile egg height from the candling stage and operational procedure of its sub-modules according to claim 1, wherein the database sub-module (m45) in the form of a database (d45) has an operating procedure **characterized in that** the application software (d42) sends data from each niche (Ni) of the virtual matrix of the incubation tray (Mz2) to a database sub-module (m45), which stores them in hardware (d45), where such data is sent to the matrix quality analysis module (m5).
